(19) Europäisches Patentamt
European Patent Office
Office européen des brevets

(11) Veröffentlichungsnummer: **0 214 592 B1**

(12) # EUROPÄISCHE PATENTSCHRIFT

(45) Veröffentlichungstag der Patentschrift: **14.08.91**

(21) Anmeldenummer: **86112093.9**

(22) Anmeldetag: **02.09.86**

Die Akte enthält technische Angaben, die nach dem Eingang der Anmeldung eingereicht wurden und die nicht in dieser Patentschrift enthalten sind.

(51) Int. Cl.5: **C07D 487/04**, C07D 498/04, C07D 513/04, C07D 487/10, C07D 498/10, C07D 513/10, A61K 31/41, //(C07D487/04, 235:00,209:00),(C07D498/04, 263:00,209:00),(C07D513/04, 277:00,209:00),(C07D487/10, 235:00,209:00),(C07D498/10, 263:00,209:00),(C07D513/10, 277:00,209:00)

(54) **Neue Pyrrolo-Benzimidazole, Verfahren zu ihrer Herstellung und diese Verbindungen enthaltende Arzneimittel.**

(30) Priorität: **05.09.85 DE 3531678**

(43) Veröffentlichungstag der Anmeldung:
**18.03.87 Patentblatt 87/12**

(45) Bekanntmachung des Hinweises auf die Patenterteilung:
**14.08.91 Patentblatt 91/33**

(84) Benannte Vertragsstaaten:
**AT BE CH DE FR GB IT LI LU NL SE**

(56) Entgegenhaltungen:
**EP-A- 0 161 632** **EP-A- 0 173 520**
**EP-A- 0 186 010** **EP-A- 0 189 103**
**DE-A- 3 410 168** **GB-A- 2 002 751**

(73) Patentinhaber: **BOEHRINGER MANNHEIM GMBH**
**Sandhofer Strasse 116**
**W-6800 Mannheim 31(DE)**

(72) Erfinder: **von der Saal, Wolfgang, Dr.rer.nat.**
**Neuer Burgweg 3**
**W-6940 Weinheim(DE)**
Erfinder: **Mertens, Alfred, Dr.rer.nat.**
**In der Schanz 31**
**W-6905 Schriesheim(DE)**
Erfinder: **Berger, Herbert, Dr.phil.**
**Völklinger Strasse 16**
**W-6800 Mannheim 31(DE)**

EP 0 214 592 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Müller-Beckmann, Bernd, Dr.med.vet.**
**Hochgewanne 46**
**W-6718 Grünstadt(DE)**

**Beschreibung**

Die vorliegende Erfindung betrifft neue tricyclische Verbindungen der allgemeinen Formel I:

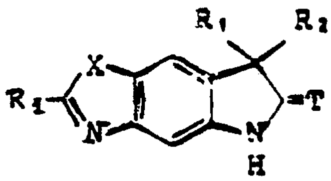

(I)

in welcher

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,

$R_2$ ein Wasserstoffatom, ein $C_1$-$C_6$ Alkyl-, $C_2$-$C_6$-Alkenyl- oder Cyangruppe, eine durch eine Hydroxy-, $C_1$-$C_6$-Alkyl-, $C_1$-$C_6$- Alkoxy-, Amino-, $C_1$-$C_6$-Alkylamino-, Di-$C_1$-$C_6$-alkylamino- oder Hydrazinogruppe substituierte Carbonylgruppe bedeutet oder mit $R_1$ zusammen eine $C_3$-$C_7$-Cycloalkylengruppe darstellt oder $R_1$ und $R_2$ zusammen eine $C_3$-$C_7$-Alkyliden- oder $C_3$-$C_7$-Cycloalkylidengruppe bilden,

T Sauerstoff oder Schwefel bedeutet,

$R_3$ ein Wasserstoffatom, die Hydroxygruppe, eine Mercapto-, $C_1$-$C_6$-Alkylmercapto-, eine Amino-, Pyridylcarbonylamino-, $C_1$-$C_6$-Alkylcarbonylaminogruppe, eine $C_1$-$C_6$-Alkyl-, $C_3$-$C_7$-Cycloalkyl-, $C_2$-$C_6$-Alkenyl-, $C_3$-$C_7$-Cycloalkenyl-, $C_2$-$C_6$-Alkinyl- oder Halogen-$C_1$-$C_6$-alkylgruppe bedeutet,

X die Gruppe -NH- darstellt,

deren Tautomere und deren physiologisch vertraegliche Salze anorganischer und organischer Saeuren und Verfahren zu ihrer Herstellung, sowie diese Verbindungen enthaltende Arzneimittel.

Verbindungen ähnlicher Strukturen sind aus den früheren Europäischen Patentanmeldungen EP-A-0 189 103, EP-A-0 161 632 und EP-A-0 186 010 bekannt.

Da die Verbindungen der allgemeinen Formel I fuer den Fall, daß $R_1$ nicht gleich $R_2$ ist, ein asymmetrisches Kohlenstoffatom besitzen, sind auch Gegenstand der Erfindung die optisch aktiven Formen und racemischen Gemische dieser Verbindungen. Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenaggregation und verbessern die Mikrozirkulation.

Die oben genannten Alkyl- oder Alkenylteile in den Resten $R_1$, $R_2$, $R_3$, $R_4$, $R_5$ können geradkettig oder verzweigt sein und 1 - 6 bzw. 2 - 6 Kohlenstoffatome und der Cycloalkylteil 3 - 7 Kohlenstoffatome enthalten.

Bevorzugt in diesem Sinne sind für $R_1$ und $R_2$ Wasserstoff, die Methyl-, Ethyl-, Isopropyl-, 3-Pentyl-, Allyl-, Cyclopentyl-, Cyclohexyl-, Cyan-, Carboxy-, Acetyl-, Propionyl-, Methoxycarbonyl-, Ethoxycarbonyl-, Aminocarbonyl-, Methylaminocarbonyl-, Dimethylaminocarbonyl- und Hydrazinocarbonylgruppe.

$R_1$ und $R_2$ koennen zusammen mit dem C-Atom, an das sie gebunden sind, auch eine Cycloalkylring mit 3-7 Kohlenstoffatomen bilden, vorzugsweise handelt es sich dabei um die Spirocyclopropyl-, Spirocyclobutyl-, Spirocyclopentyl- und Spirocyclohexylgruppe.

$R_1$ und $R_2$ koennen zusammen auch eine $C_3$-$C_7$ Alkyliden- oder $C_3$-$C_7$ Cycloalkylidengruppe bilden, vorzugsweise handelt es sich dabei um die Isopropyliden- oder Cyclohexylidengruppe.

In der allgemeinen Formel I kann der Substituent $R_3$ Wasserstoff, eine geradkettige oder verzweigte Alkylgruppe mit 1-6 Kohlenstoffatomen, eine geradkettige oder verzweigte Alkenyl- oder Alkinylgruppe mit 2-6 Kohlenstoffatomen, eine Cycloalkyl- oder Cycloalkenylgruppe mit 3-6 Kohlenstoffatomen oder eine Halogenalkylgruppe mit 1-6 Kohlenstoffatomen sein. Bevorzugt in diesem Sinne sind die Methyl-, Ethyl-, Propyl-, Butyl-, Pentyl-, Hexyl-, Vinyl-, Propenyl-, Butenyl-, Pentenyl-, Propinyl-, Butinyl-, Cyclopropyl-, Cyclobutyl-, Cyclopentyl-, Cyclohexyl-, Cyclopentenyl-, Cyclohexenyl-, Trifluormethyl-, Pentafluorethyl- und Heptafluorpropylgruppe.

Der Substituent $R_3$ kann auch die Hydroxy-, die Mercapto-, die Amino-, die Pyridylcarbonylamino-, die Alkylmercapto- oder die Alkylcarbonylamino gruppe sein. Die Alkylketten koennen geradkettig oder verzweigt sein und 1-6 Kohlenstoffatome enthalten. Bevorzugt in diesem Sinne sind die Methylmercapto-, Ethylmercapto-, Propylmercapto-, Butylmercapto-, Acetylamino-, Propionylaminogruppe.

Besonders bevorzugte Verbindungen sind Verbindungen der allgemeinen Formel I

in der

$R_1$ und $R_2$ gleich sind und die Methyl- oder Ethylgruppe darstellen,

$R_1$ und $R_2$ verschieden sind und Wasserstoff, die Methyl-, Ethyl-, Isopropyl-, Cyclopentyl-, Cyan-, Acetyl-, Methoxy-carbonyl-, Ethoxycarbonyl-, Aminocarbonyl- und Hydrazinocarbonylgruppe bedeuten,
$R_1$ und $R_2$ einen Spirocyclopentylring darstellen, wenn $R_1$ und $R_2$ mit dem C-Atom, an das sie gebunden sind, einen Cycloalkylring bilden,
$R_3$ ein Wasserstoffatom, die Methyl-, Ethyl-, Propyl-, Isopropyl-, Butyl-, Tert.-butyl-, Pentyl-, Hexyl-, Propenyl-, Isopropenyl-, Cyclopropyl-, Cyclopentenyl-, Cyclohexyl-, Trifluormethyl-, Hydroxy-, Mercapto-, Methylmercapto-, Amino-, Acetylamino- und Pyridylcarbonylamino gruppe darstellt,
X die Gruppe -NH- und

T ein Sauerstoffatom darstellen.

Die Verbindungen der allgemeinen Formel I und deren Tautomere koennen nach den in Schemata 1-6 gezeigten Verfahren hergestellt werden.

1. Verbindungen der allgemeinen Formel I', und deren Tautomere, in der $R_1$, $R_2$ und T die oben angegebenen Bedeutungen besitzen und in der $R_3$' ein Wasserstoffatom, eine Alkyl-, Cycloalkyl-, Alkenyl-, Cycloalkenyl-, Alkinyl- oder Halogenalkyl gruppe bedeutet, erhaelt man, wenn man, wie in Schema 1 gezeigt, Amino-nitro-indol-derivate der allgemeinen Formel II, in der $R_1$, $R_2$ und T die oben angegebenen Bedeutungen besitzen, mit Verbindungen der allgemeinen Formel III acyliert, und die dabei erhaltenen Amide der allgemeinen Formel IV reduziert und cyclisiert. In den Verbindungen der allgemeinen Formel III hat $R_3$' die oben angegebenen Bedeutungen und Y stellt die Hydroxygruppe oder einen leicht abspaltbaren Rest dar. In den Verbindungen der allgemeinen Formel IV haben $R_1$, $R_2$, T und $R_3$' die oben angegebenen Bedeutungen.

Schema 1

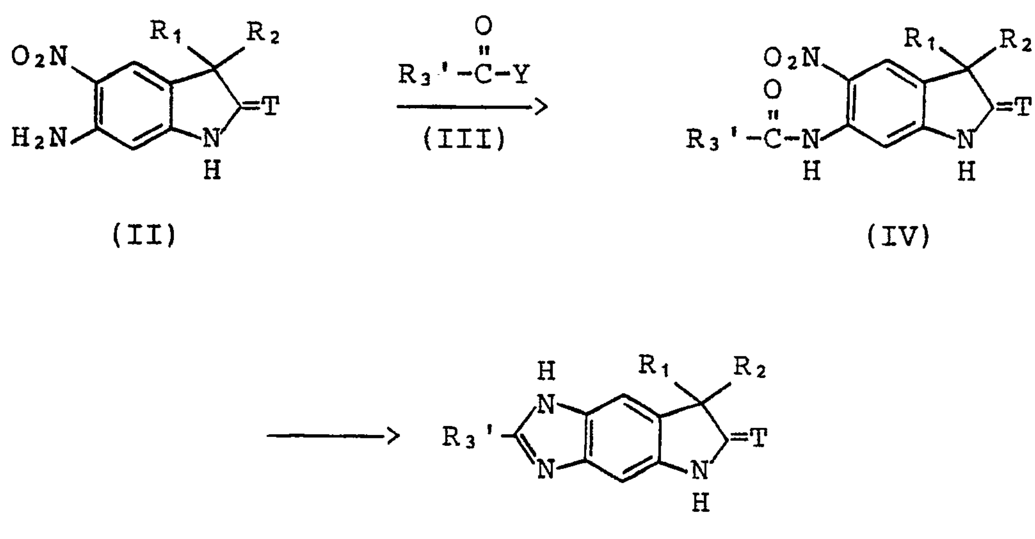

(II)

(III)

(IV)

(I')

2. In genau der gleichen Weise erhaelt man die Verbindungen der allgemeinen Formel I", in der $R_1$, $R_2$, $R_3$', X und T die oben angegebenen Bedeutungen haben, wie in Schema 2 gezeigt, indem man Indolderivate der allgemeinen Formel V durch Verbindungen der allgemeinen Formel III acyliert, und anschließend die so erhaltenen Verbindungen der allgemeinen Formel VI reduziert und cyclisiert. In den Formeln III, V und VI haben $R_1$, $R_2$, $R_3$', X, T und Y die oben angegebenen Bedeutungen.

Schema 2

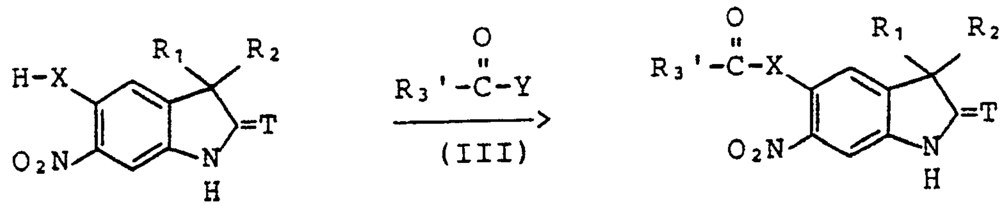


(V) (VI)

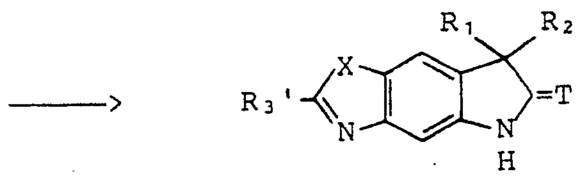

(I")

3. Wie in Schema 3 gezeigt, kann man die Verbindungen der allgemeinen Formel I", in der $R_1$, $R_2$, $R_3$', X und T die oben angegebenen Bedeutungen besitzen, auch erhalten, indem man die Aminoindolderivate der allgemeinen Formel VII, in der $R_1$, $R_2$, T und X die oben angegebenen Bedeutungen besitzen, mit Verbindungen der allgemeinen Formel VIII acyliert und cyclisiert. In der allgemeinen Formel VIII hat $R_3$' die oben angegebenen Bedeutungen und Z stellt Wasserstoff, die Hydroxygruppe oder einen leicht abspaltbaren Rest dar.

Schema 3

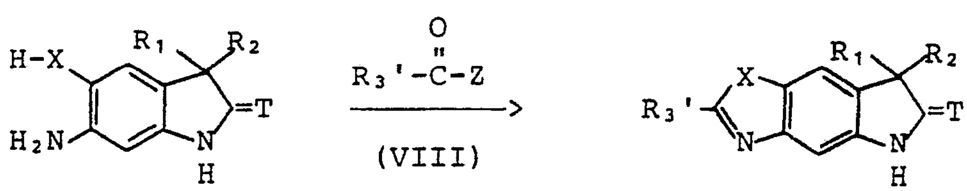


(VII) (I")

Unter Verbindungen der allgemeinen Formeln III und VIII versteht man Carbonsaeuren, Saeurehalogenide wie Saeurechloride, Carbonsaeureester wie Methyl- und Ethylester und andere aktivierte Carbonsaeurederivate wie z.B. Anhydride. Daneben stellen Verbindungen der allgemeinen Formel VIII auch Aldehyde dar.

Sind die Verbindungen der allgemeinen Formeln III und VIII Carbonsaeuren, so wird die Acylierung der Verbindungen der allgemeinen Formeln II, V oder VII in Gegenwart eines wasserentziehenden Stoffes wie etwa N,N'-Dicyclohexylcarbodiimid und in einem neutralen Loesungsmittel wie Methylenchlorid bei Temperaturen von -50° C bis +100° C, vorzugsweise bei Raumtemperatur, oder in Gegenwart von Polyphosphorsaeure bei Temperaturen von 50° C bis 250° C, vorzugsweise zwischen 100° C und 200° C vorgenommen.

Ist die Verbindung der allgemeinen Formel III oder VIII ein Carbonsaeurederivat, so findet die Umsetzung mit Verbindungen der allgemeinen Formel II, V oder VII in inerten Loesungsmitteln, vorzugsweise in Methylenchlorid oder Pyridin statt. Die Reduktion der Nitrogruppe in Verbindungen der allgemeinen Formel IV oder VI erfolgt durch Hydrierung in einem Loesungsmittel wie Wasser, Ethanol, Eisessig, Essigsaeureethylester oder Dimethylformamid zweckmaeßigerweise mit Wasserstoff in Gegenwart eines Hydrierungskatalysators wie Raney-Nickel, Platin oder Palladium auf Kohle. Die Reduktion ist

auch moeglich durch Metalle wie Eisen, Zinn oder Zink in Gegenwart einer Saeure, mit Salzen wie Eisen-II-sulfat, Zinn-II-chlorid, Natriumsulfid, Natriumhydrogensulfid oder Natriumdithionit, oder mit Hydrazin in Gegenwart von Raney-Nickel. Die Reaktionen werden bei 0 bis 250° C, vorzugsweise bei Raumtemperatur durchgefuehrt.

Die Cyclisierung zu Verbindungen der allgemeine Formel I' oder I" erfolgt bereits unter den Bedingungen der oben erwaehnten Reduktion der Nitrogruppe in Verbindungen der allgemeinen Formel IV oder VI. Gewuenschtenfalls kann diese Cyclisierung vervollstaendigt werden, indem man das Reaktionsprodukt aus der Reduktion der Nitrogruppe in einem Loesungsmittel oder Loesungsmittelgemisch wie Ethanol, Isopropanol, Eisessig, Benzol, Chlorbenzol, Glycol, Diethylenglycoldimethylether, Sulfolan oder Dimethylformamid auf Temperaturen zwischen 0° und 250° C, vorzugsweise auf die Siedetemperatur des Loesungsmittel erhitzt, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxyd, Kaliummethylat oder Kalium-tert.-butylat durchfuehrt. Die Cyclisierung kann jedoch auch ohne Loesungsmittel und/oder Kondensationsmittel durchgefuehrt werden. Erfolgte die Umsetzung jedoch nach Schema 3, so sind die erwaehnten Bedingungen zur Cyclisierung unbedingt notwendig.

Sind Verbindungen der allgemeinen Formel VIII Aldehyde, so findet die Umsetzung zur Schiffschen Base vorzugsweise in alkoholischem Medium statt, die anschließende Cyclisierung und Oxidation erfolgt durch Erwaermen des Reaktionsansatzes zum Rueckfluß in Gegenwart von Luftsauerstoff und katalytischen Mengen Saeure, wie Z.B. Essigsaeure oder Toluolsulfonsaeure und gegebenenfalls katalytischer Mengen Mangandioxid.

Schema 4

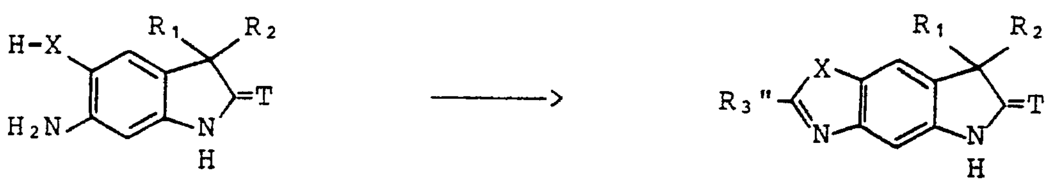

(VII) (I"')

4. Wie aus Schema 4 ersichtlich, lassen sich Verbindungen der allgemeinen Formel VII auch zu Verbindungen der allgemeinen Formel I"' cyclisieren, wobei $R_1$, $R_2$, T und X die oben angegebenen Bedeutungen haben und $R_3$" die Hydroxy-, Mercapto- oder Aminogruppe bedeutet. Dies geschieht durch Umsetzung mit Reagenzien, die Carbonylgruppen oder deren Aequivalente wie Thiocarbonyl- und Iminogruppen uebertragen, vorzugsweise mit Phosgen, 1,1'-Carbonyldiimidazol, Thiophosgen oder Bromcyan.

Diese Reaktionen werden in einem Loesungsmittel oder Loesungsmittelgemisch wie Benzol, Toluol, Chlorbenzol, Dimethylformamid oder waessrige Salzsaeure bei Temperaturen zwischen -20° C und 100° C, vorzugsweise bei Raumtemperatur vorgenommen.

5. Verbindungen der allgemeinen Formel I"", in der $R_1$, $R_2$, T und X die angegebenen Bedeutungen haben, lassen sich herstellen, indem man Verbindungen der allgemeinen Formeln II oder V mit Halogenameisensaeureestern, vorzugsweise mit Chlorameisensaeureethylester, umsetzt, anschließend die Nitrogruppe reduziert und cyclisiert. Diese Reduktion und die Cyclisierung erfolgen unter den oben beschriebenen Bedingungen.

Schema 5

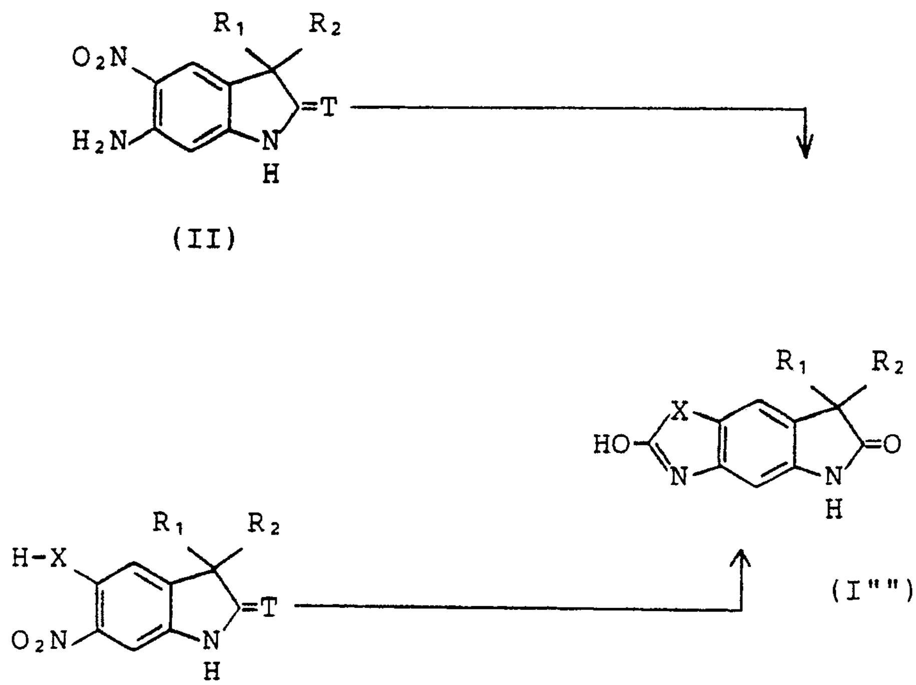

(II)

(V)

(I"")

Schema 6

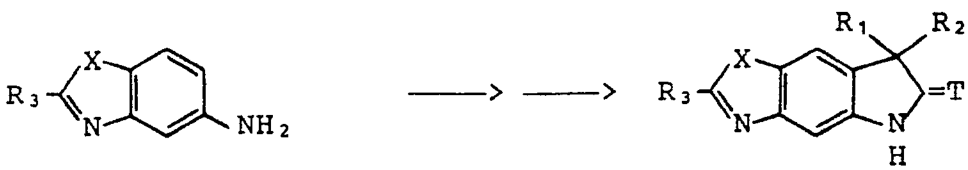

(IX) (I)

6. Verbindungen der allgemeinen Formel IX lassen sich, wie in Schema 6 gezeigt, nach literaturbekannten Verfahren zu Verbindungen der allgemeinen Formel I cyclisieren, wobei $R_1$, $R_2$, $R_3$, T und X die oben angegebenen Bedeutungen haben. Dies geschieht mit Bisulfit-Additionsverbindungen entsprechender Ketone (Hinsberg-Synthese) oder ueber entsprechende Hydrazide (Brunner-Synthese) oder ueber entsprechende Amide (Stollé-Synthese). Vergleiche hierzu: R.C. Elderfield (Herausgeber), P.L. Julian, E.W. Meyer und H.C. Printy in "Heterocyclic Compounds", Band 3, S. 126-186, J. Wiley and Sons 1952, New York.

Eine erfindungsgemaeß erhaltene Verbindung der allgemeinen Formel I oder deren Tautomer kann gewuenschtenfalls auch in eine andere Verbindung der allgemeinen Formel I umgewandelt werden. Das trifft z.B. zu fuer

a) die Umwandlung von Verbindungen der allgemeinen Formel I in denen $R_1 = R_2 =$ Wasserstoff bedeuten zu Verbindungen der allgemeinen Formel I in denen $R_1$ zusammen mit $R_2$ die Isopropylidengruppe, die Cyclopentyliden- oder Cyclohexylidengruppe darstellen, sowie gegebenenfalls deren Hydrierung zu den entsprechenden Verbindungen der allgemeinen Formel I, in denen $R_1$ oder $R_2$ gleich Wasserstoff ist.

Dies betrifft z.B. die Umsetzung mit Verbindungen der allgemeinen Formel X

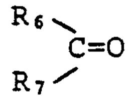

(X) ,

in welcher $R_6$ und $R_7$ Alkylgruppen sind oder $R_6$ und $R_7$ zusammen eine $C_3$-$C_7$-Cycloalkylengruppe bilden, in Gegenwart einer Base wie Ammoniak oder Triethylamin in alkoholischer Loesung.

b) die nachtraegliche Umwandlung von Verbindungen der allgemeinen Formel I in denen $R_1$ oder $R_2$ eine Carboxylgruppe oder ein reaktionsfaehiges Derivat wie z.B. Carbonsaeureester oder Saeurechlorid darstellt, mit Hydrazin, Ammoniak, einem primaeren oder sekundaeren Amin oder einem reaktionsfaehigen Derivat hiervon zu neuen Verbindungen der allgemeinen Formel I, in denen $R_1$ oder $R_2$ eine durch eine Amino-, Alkylamino-, Dialkylamino- oder Hydrazinogruppe substituierte Carbonylgruppe ist. Die nachtraegliche Umwandlung betrifft auch Verbindungen der allgemeinen Formel I in denen $R_1$ oder $R_2$ eine Aminocarbonylgruppe darstellt zu solchen in denen $R_1$ oder $R_2$ eine Cyangruppe ist, sowie die nachtraegliche Umwandlung einer Cyanogruppe in eine Carboxyl-, Aminocarbonyl- oder Alkoxycarbonylgruppe.

Die Reaktion von Carbonsaeurederivaten mit Aminderivaten wird zweckmaeßigerweise in einem Loesungsmittel oder Loesungsmittelgemisch durchgefuehrt, wie Methylenchlorid Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Saeure aktivierenden Mittels oder eines wasserentziehenden Mittels, z.B. in Gegenwart von Chlorameisensaeureethylester, Thionylchlorid, Phoshortrichlorid, Phosphorpentoxid, N'N-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Hydrazino- oder Aminogruppe aktivierender Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen -25 und 250° C, vorzugsweise jedoch bei Temperaturen zwischen -10° C und der Siedetemperatur des verwendeten Loesungsmittels. Des weiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsaeure- oder Sulfonsaeurechlorid, und Hydrazin oder einem entsprechenden Amin, wobei diese gleichzeitig als Loesungsmittel dienen koennen, und bei Temperaturen zwischen 0 und 50° C durchgefuehrt.

Die Umsetzung zur Cyangruppe wird zweckmaeßigerweise in einem inerten Loesungsmittel wie z.B. in Methylenchlorid, Chloroform, Dioxan, Pyridin, Xylol, Chlorbenzol in Gegenwart eines wasserentziehenden Mittels wie z.B. Thionylchlorid, Phosphortrichlorid, Phosphorpentoxid, Phosphorpentachlorid, Aluminiumchlorid, Benzolsulfonsaeurechlorid, Toluolsulfonsaeurechlorid, Triphenylphosphin, Bortrifluorid oder Polyphosphorsaeureethylester bei Temperaturen zwischen 50 und 250° vorzugsweise jedoch bei der Siedetemperatur des Loesungsmittels durchgefuehrt.

Erhaelt man eine Verbindung der allgemeinen Formel I, in der $R_2$ die Cyangruppe darstellt, so kann diese anschließend mittels Alkoholyse und/oder Hydrolyse in eine entsprechende Verbindung, in der $R_2$ eine Alkoxycarbonylgruppe mit insgesamt 2-5 Kohlenstoffatomen, die Aminocarbonyl- oder Carboxylgruppe ueberfuehrt werden und/oder eine Verbindung der allgemeinen Formel I, in der $R_2$ die Carboxygruppe darstellt, mittels Veresterung in eine entsprechende Verbindung der allgemeinen Formel 1, in der $R_2$ eine Alkoxycarbonylgruppe mit insgesamt 2-5 Kohlenstoffatomen darstellen, ueberfuehrt werden.

Die nachtraegliche Alkoholyse und/oder Hydrolyse wird zweckmaeßigerweise entweder in Gegenwart einer Saeure wie Salzsaeure, Schwefelsaeure, Phosphorsaeure oder Trichloressigsaeure oder in Gegenwart einer Base wie Natriumhydroxid oder Kaliumhydroxid in einem geeigneten Loesungsmittel wie Wasser, Wasser/Methanol, Ethanol, Wasser/Ethanol, Wasser/Isopropanol oder Wasser/Dioxan bei Temperaturen zwischen -10 und 120° C, z.B. bei Temperaturen zwischen Raumtemperatur und der Siedetemperatur des Reaktionsgemisches, durchgefuehrt.

Die nachtraegliche Veresterung wird zweckmaeßigerweise in einem geeignten Loesungsmittel, z.B. in einem entsprechenden Alkohol, Pyridin, Toluol, Methylenchlorid, Tetrahydrofuran oder Dioxan, in Gegenwart eines saeureaktivierenden und/oder wasserentziehenden Mittels wie Thionylchlorid, Chlorameisensaeureethylester, N,N'-Carbonyldiimidazol oder N,N'-Dicyclohexylcarbodiimid oder dessen Isoharnstoffethern, gegebenenfalls in Gegenwart eines Reaktionsbeschleunigers wie Kupfer-chlorid, oder durch Umesterung, z.B. mit einem entsprechenden Kohlensaeurediester, bei Temperaturen zwischen 0 und 100° C, vorzugsweise jedoch bei Temperaturen zwischen 20° C und der Siedetemperatur des betreffenden Loesungsmittels, durchgefuehrt.

c) Diese Veresterungsbedingungen lassen sich auch anwenden, um Verbindungen der allgemeinen Formel I, in denen $R_1$, $R_2$, T und X die oben angegebenen Bedeutungen besitzen und $R_3$ ein Carboxyalkylrest darstellt, in andere Verbindungen der allgemeinen Formel I umzuwandeln, in denen $R_3$ einen Alkoxycarbonylalkylrest darstellt.

d) Verbindungen der allgemeinen Formel I, in denen $R_1$, $R_3$, T und X die oben angegebenen Bedeutungen besitzen und $R_2$ einen Alkoxycarbonylrest darstellt, lassen sich in Verbindungen der allgemeinen Formel I umwandeln, in denen $R_2$ ein Wasserstoffatom darstellt. Dies geschieht durch Verseifung des Alkoxycarbonylrestes und Decarboxylierung. Die Verseifung wird zweckmaeßigerweise in Gegenwart einer Saeure wie Salzsaeure, Schwefelsaeure, Phosphorsaeure oder Trichloressigsaeure oder in Gegenwart einer Base wie Natriumhydroxid oder Kalimhydroxid in einem geeigneten Loesungsmittel wie Wasser, Wasser/Dioxan bei Temperaturen zwischen 0° C und 120° C, vorzugsweise bei der Siedetemperatur des Reaktionsgemisches durchgefuehrt. Die Decarboxylierung tritt dabei meist spontan ein. Sie kann gewuenschtenfalls durch Erhoehung der Reaktionstemperatur beschleunigt werden.

e) die nachtraegliche Umwandlung zu Verbindungen der allgemeinen Formel I und den zu den Verbindungen der allgemeinen Formel I fuehrenden Zwischenprodukten in denen T ein Schwefelatom bedeutet, aus solchen in denen T ein Sauerstoffatom darstellt. Sie wird nach literaturbekannten Verfahren mit einem das Schwefelatom uebertragenden Reagenz wie z.B. Phosphorpentasulfid oder 2,4-Bis(4-methoxyphenyl)-2,4-dithioxo-1,3,2,4-dithiadiphosphetan in einem geeigneten Loesungsmittel wie z.B. Tetrahydrofuran, Dioxan, Ethylenglycoldimethylether, Benzol, Toluol oder Pyridin bei Temperaturen zwischen 0° C und der Siedetemperatur des Reaktionsgemisches durchgefuehrt.

f) die nachtraegliche Alkylierung von Verbindungen der allgemeinen Formel I, in der $R_3$ die Mercaptogruppe darstellt, zu Verbindungen der allgemeinen Formel I, in der $R_3$ die Alkylmercaptogruppe darstellt.

Die Reaktionen werden vorzugsweise in einem Loesungsmittel wie Aceton, Ether, Benzol, Toluol, Dimethylformamid bei Temperaturen zwischen -30° C und +100° C, vorzugsweise bei Raumtemperatur, in Gegenwart einer Base wie Kaliumcarbonat oder Natriumhydrid und eines Alkylierungsmittels wie Alkylhalogeniden oder Alkylsulfaten durchgefuehrt.

g) die nachtraegliche Acylierung von Verbindungen der allgemeinen Formel I, in der $R_3$ die Aminogruppe bedeutet zu Verbindungen der allgemeinen Formel I in der $R_3$ die Acylaminogruppe bedeutet. Diese Reaktionen werden vorzugsweise durch Umsetzung mit Carbonsaeurederivaten wie Saeurehalogeniden, Carbonsaeureestern oder anderen aktivierten Carbonsaeurederivaten wie z.B. Anhydriden, durchgefuehrt.

Die Verbindungen der allgemeinen Formel I, in der $R_1$, $R_2$, $R_3$ und T die oben genannten Bedeutungen besitzen, und deren Tautomere koennen gewuenschtenfalls in ihre pharmakologisch unbedenklichen Salze ueberfuehrt werden.

Dazu setzt man diese vorzugsweise in einem organischen Loesungsmittel mit der aequivalenten Menge einer anorganischen oder organischen Saeure, z.B. Salzsaeure, Bromwasserstoffsaeure, Phosphorsaeure, Schwefelsaeure. Essigsaeure, Citronensaeure, Weinsaeure, Maleinsaeure, Fumarsaeure, Benzoesaeure oder Cyclohexylsulfaminsaeure um.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden.Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komblexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Sterinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 0.1-500 mg pro Tag bezogen auf 75 kg Koerpergewicht appliziert. Bevorzugt ist es, 2-3 mal taeglich 1-2 Tabletten mit einem Wirkstoffgehalt von 0.1-200mg zu verabreichen. Die Tabletten koennen auch retardiert sein, wodurch nur noch einmal am Tag 1-2 Tabletten mit 0.1-500 mg Wirkstoff gegeben werden muß. Der Wirkstoff kann auch durch Injektion 1-8 mal pro Tag bzw. durch Dauerfusion gegeben werden, wobei Mengen von 0.1-200 mg pro Tag normalerweise ausreichen.

Bevorzugt im Sinne der vorliegenden Erfindung sind außer den in den Beispielen genannten Verbindungen die folgenden und deren Tautomere:

2-Methyl-6,7-dihydro-3H,5H-pyrrolo[2,3-f]benzimidazol-6-on
7-Acetyl-2-methyl-6,7-dihydro-3H,5H-pyrrolo[2,3-f]benzimidazol-6-on
7-Acetyl-2,7-dimethyl-6,7-dihydro-3H,5H-pyrrolo[2,3-f]benzimidazol-6-on
7-Allyl-2-methyl-6,7-dihydro-3H,5H-pyrrolo[2,3-f]benzimidazol-6-on
7-Aminocarbonyl-2,7-dimethyl-6,7-dihydro-3H,5H-pyrrolo[2,3-f]benzimidazol-6-on
7-Cyan-2,7-dimethyl-6,7-dihydro-3H,5H-pyrrolo[2,3-f]benzimidazol-6-on
7-Cyclohexyl-2-methyl-6,7-dihydro-3H,5H-pyrrolo[2,3-f]benzimidazol-6-on
7,7-Diallyl-2-methyl-6,7-dihydro-3H,5H-pyrrolo[2,3-f]benzimidazol-6-on
7,7-Diethyl-2-methyl-6,7-dihydro-3H,5H-pyrrolo[2,3-f]benzimidazol-6-on
7-Ethoxycarbonyl-2-methyl-6,7-dihydro-3H,5H-pyrrolo[2,3-f]benzimidazol-6-on
7-Ethoxycarbonyl-2,7-dimethyl-6,7-dihydro-3H,5H-pyrrolo[2,3-f]benzimidazol-6-on
7-Hydrazinocarbonyl-2,7-dimethyl-6,7-dihydro-3H,5H-pyrrolo[2,3-f]benzimidazol-6-on
7-Isopropyliden-2-methyl-6,7-dihydro-3H,5H-pyrrolo[2,3-f]benzimidazol-6-on
7-Methoxycarbonyl-2-methyl-6,7-dihydro-3H,5H-pyrrolo[2,3-f]benzimidazol-6-on
7-Methoxycarbonyl-2,7-dimethyl-6,7-dihydro-3H,5H-pyrrolo[2,3-f]benzimidazol-6-on
2,7-Dimethyl-6,7-dihydro-3H,5H-pyrrolo[2,3-f]benzimidazol-6-on
7-(2-Methyl-propyl)-2-methyl-6,7-dihydro-3H,5H-pyrrolo[2,3-f]benzimidazol-6-on
7-(3-Pentyl)-2-methyl-6,7-dihydro-3H,5H-pyrrolo[2,3-f]benzimidazol-6-on
7-(2-Propyl)-2-methyl-6,7-dihydro-3H,5H-pyrrolo[2,3-f]benzimidazol-6-on
2'-Methyl-spiro[cyclopentan-1,7'-6',7'-dihydro-3'H,5'H-pyrrolo[2',3'-f]benzimidazol]-6'-on
7-Acetyl-7-methyl-2-hydroxy-6,7-dihydro-3H,5H-pyrrolo[2,3-f]benzimidazol-6-on
7-Propyl-2-hydroxy-6,7-dihydro-3H,5H-pyrrolo[2,3-f]benzimidazol-6-on
7-Aminocarbonyl-2-hydroxy-6,7-dihydro-3H,5H-pyrrolo[2,3-f]benzimidazol-6-on
7-Cyan-2-hydroxy-6,7-dihydro-3H,5H-pyrrolo[2,3-f]benzimidazol-6-on
7-Ethoxycarbonyl-2-hydroxy-6,7-dihydro-3H,5H-pyrrolo[2,3-f]benzimidazol-6-on
7-Hydrazincarbonyl-2-hydroxy-6,7-dihydro-3H,5H-pyrrolo[2,3-f]benzimidazol-6-on
7-Isopropyliden-2-hydroxy-6,7-dihydro-3H,5H-pyrrolo[2,3-f]benzimidazol-6-on
7-Methoxycarbonyl-7-methyl-2-hydroxy-6,7-dihydro-3H,5H-pyrrolo[2,3-f]benzimidazol-6-on
7-(2-Propyl)-2-hydroxy-6,7-dihydro-3H,5H-pyrrolo[2,3-f]benzimidazol-6-on
2'-Hydroxy-spiro[cyclopentan-1,7'-6',7'-dihydro-3'H,5'H-pyrrolo[2',3'-f]benzimidazol]-6'-on
7-Acetyl-7-methyl-6,7-dihydro-3H,5H-pyrrolo[2,3-f]benzimidazol-6-on
7-Ethoxycarbonyl-7-methyl-6,7-dihydro-3H,5H-pyrrolo[2,3-f]benzimidazol-6-on
7-Acetyl-7-methyl-2-propyl-6,7-dihydro-3H,5H-pyrrolo[2,3-f]benzimidazol-6-on
7-Ethyl-2-propyl-6,7-dihydro-3H,5H-pyrrolo[2,3-f]benzimidazol-6-on
7-Ethoxycarbonyl-7-methyl-2-propyl-6,7-dihydro-3H,5H-pyrrolo[2,3-f]benzimidazol-6-on
7-Acetyl-7-methyl-2-(2-propyl)-6,7-dihydro-3H,5H-pyrrolo[2,3-f]benzimidazol-6-on
7-Methyl-2-(2-propyl)-6,7-dihydro-3H,5H-pyrrolo[2,3-f]benzimidazol-6-on
7-Ethyl-2-(2-propyl)-6,7-dihydro-3H,5H-pyrrolo[2,3-f]benzimidazol-6-on
7-Ethoxycarbonyl-7-methyl-2-(2-propyl)-6,7-dihydro-3H,5H-pyrrolo[2,3-f]benzimidazol-6-on
2'-(2-Propyl)-spiro[cyclopentan-1,7'-6',7'-dihydro-3'H,5'H-pyrrolo[2',3'-f]benzimidazol]-6'-on
7-Acetyl-7-methyl-2-cyclohexyl-6,7-dihydro-3H,5H-pyrrolo[2,3-f]benzimidazol-6-on
7-Methyl-2-cyclohexyl-6,7-dihydro-3H,5H-pyrrolo[2,3-f]benzimidazol-6-on
7-Ethyl-2-cyclohexyl-6,7-dihydro-3H,5H-pyrrolo[2,3-f]benzimidazol-6-on
7-Ethoxycarbonyl-7-methyl-2-cyclohexyl-6,7-dihydro-3H,5H-pyrrolo[2,3-f]benzimidazol-6-on
2'-Cyclohexyl-spiro[cyclopentan-1,7'-6',7'-dihydro-3'H,5'H, pyrrolo[2',3'-f]benzimidazol]-6'-on
7-Acetyl-7-methyl-2-(2-propenyl)-6,7-dihydro-3H,5H-pyrrolo[2,3-f]benzimidazol-6-on
7-Methyl-2-(2-propenyl)-6,7-dihydro-3H,5H-pyrrolo[2,3-f]benzimidazol-6-on
7-Ethyl-2-(2-propenyl)-6,7-dihydro-3H,5H-pyrrolo[2,3-f]benzimidazol-6-on
7-Ethoxycarbonyl-7-methyl-2-(2-propenyl)-6,7-dihydro-3H,5H-pyrrolo[2,3-f]benzimidazol-6-on
2'-(2-Propenyl)-spiro[cyclopentan-1,7'-6',7'-dihydro-3'H,5'H-pyrrolo[2',3'-f]benzimidazol]-6'-on

7-Acetyl-7-methyl-2-(1-cyclopentenyl)-6,7-dihydro-3H,5H-pyrrolo[2,3-f]benzimidazol-6-on
7-Methyl-2-(1-cyclopentenyl)-6,7-dihydro-3H,5H-pyrrolo[2,3-f]benzimidazol-6-on
7-Ethyl-2-(1-cyclopentenyl)-6,7-dihydro-3H,5H-pyrrolo[2,3-f]benzimidazol-6-on
7-Ethoxycarbonyl-7-methyl-2-(1-cyclopentenyl)-6,7-dihydro-3H,5H-pyrrolo[2,3-f]benzimidazol-6-on
2'-(1-Cyclopentenyl)-spiro[cyclopentan-1,7'-6',7'-dihydro-3'H,5'H-pyrrolo[2',3'-f]benzimidazol]-6'-on
7-Acetyl-7-methyl-2-trifluormethyl-6,7-dihydro-3H,5H-pyrrolo[2,3-f]benzimidazol-6-on
7-Methyl-2-trifluormethyl-6,7-dihydro-3H,5H-pyrrolo-[2,3-f]benzimidazol-6-on
7-Ethyl-2-trifluormethyl-6,7-dihydro-3H,5H-pyrrolo[2,3-f]benzimidazol-6-on
7-Ethoxycarbonyl-7-methyl-2-trifluormethyl-6,7-dihydro-3H,5H-pyrrolo[2,3-f]benzimidazol-6-on
2'-Trifluormethyl-spiro[cyclopentan-1,7'-6',7'-dihydro-3'H, 5'H-pyrrolo[2',3'-f]benzimidazol]-6'-on
7-Acetyl-7-methyl-2-mercapto-6,7-dihydro-3H,5H-pyrrolo[2,3-f]benzimidazol-6-on
7-Methyl-2-mercapto-6,7-dihydro-3H,5H-pyrrolo[2,3-f]benzimidazol-6-on
7-Ethyl-2-mercapto-6,7-dihydro-3H,5H-pyrrolo[2,3-f]benzimidazol-6-on
7-Ethoxycarbonyl-7-methyl-2-mercapto-6,7-dihydro-3H,5H-pyrrolo[2,3-f]benzimidazol-6-on
2'-Mercapto-spiro[cyclopentan-1,7'-6',7'-dihydro-3'H,5'H-pyrrolo[2',3'-f]benzimidazol]-6'-on
7-Acetyl-7-methyl-2-amino-6,7-dihydro-3H,5H-pyrrolo[2,3-f]benzimidazol-6-on
7-Methyl-2-amino-6,7-dihydro-3H,5H-pyrrolo[2,3-f]benzimidazol-6-on
7-Ethyl-2-amino-6,7-dihydro-3H,5H-pyrrolo[2,3-f]benzimidazol-6-on
7-Ethoxycarbonyl-7-methyl-2-amino-6,7-dihydro-3H,5H-pyrrolo[2,3-f]benzimidazol-6-on
2'-Amino-spiro[cyclopentan-1,7'-6',7'-dihydro-3'H,5'H-pyrrolo[2',3'-f]benzimidazol]-6'-on
7-Acetyl-7-methyl-2-acetamino-6,7-dihydro-3H,5H-pyrrolo[2,3-f]benzimidazol-6-on
7-Methyl-2-acetamino-6,7-dihydro-3H,5H-pyrrolo[2,3-f]benzimidazol-6-on
7-Ethyl-2-acetamino-6,7-dihydro-3H,5H-pyrrolo[2,3-f]benzimidazol-6-on
7-Ethoxycarbonyl-7-methyl-2-acetamino-6,7-dihydro-3H,5H-pyrrolo[2,3-f]benzimidazol-6-on
2'-Acetamino-spiro[cyclopentan-1,7'-6',7'-dihydro-3'H,5'H-pyrrolo[2',3'-f]benzimidazol]-6'-on
7,7-Dimethyl-2-acetamino-6,7-dihydro-3H,5H-pyrrolo[2,3-f]benzimidazol-6-on
7-Acetyl-7-methyl-2-methylmercapto-6,7-dihydro-3H,5H-pyrrolo[2,3-f]benzimidazol-6-on
7-Methyl-2-methylmercapto-6,7-dihydro-3H,5H-pyrrolo[2,3-f]benzimidazol-6-on
7-Ethyl-2-methylmercapto-6,7-dihydro-3H,5H-pyrrolo[2,3-f]benzimidazol-6-on
7-Ethoxycarbonyl-7-methyl-2-methylmercapto-6,7-dihydro-3H,5H-pyrrolo[2,3-f]benzimidazol-6-on
2'-Methylmercapto-spiro[cyclopentan-1,7'-6',7'-dihydro-3'H, 5'H-pyrrolo[2',3'-f]benzimidazol]-6'-on
7,7-Dimethyl-2-ethyl-6,7-dihydro-3H,5H-pyrrolo[2,3-f]benzimidazol-6-on
7,7-Dimethyl-2-(1-butyl)-6,7-dihydro-3H,5H-pyrrolo[2,3-f]benzimidazol-6-on
7,7-Dimethyl-2-(2-butyl)-6,7-dihydro-3H,5H-pyrrolo[2,3-f]benzimidazol-6-on
7,7-Dimethyl-2-ethenyl-6,7-dihydro-3H,5H-pyrrolo[2,3-f]benzimidazol-6-on
7,7-Dimethyl-2-(1,2-dimethyl-ethenyl)-6,7-dihydro-3H,5H-pyrrolo[2,3-f]benzimidazol-6-on
7,7-Dimethyl-2-(2,2-dimethyl-ethenyl)-6,7-dihydro-3H,5H-pyrrolo[2,3-f]benzimidazol-6-on
7,7-Dimethyl-2-(1-butenyl)-6,7-dihydro-3H,5H-pyrrolo[2,3-f]benzimidazol-6-on
7,7-Dimethyl-2-(4-butenyl)-6,7-dihydro-3H,5H-pyrrolo[2,3-f]benzimidazol-6-on
7,7-Dimethyl-2-(1-hexenyl)-6,7-dihydro-3H,5H-pyrrolo[2,3-f]benzimidazol-6-on
7,7-Dimethyl-2-(2-hexenyl)-6,7-dihydro-3H,5H-pyrrolo[2,3-f]benzimidazol-6-on
7,7-Dimethyl-2-(1-methyl-1-butenyl)-6,7-dihydro-3H,5H-pyrrolo[2,3-f]benzimidazol-6-on
7,7-Dimethyl-2-cyclobutyl-6,7-dihydro-3H,5H-pyrrolo[2,3-f}benzimidazol-6-on
7,7-Dimethyl-2-cyclopentyl-6,7-dihydro-3H,5H-pyrrolo[2,3-f]benzimidazol-6-on
7,7-Dimethyl-2-cyclohexenyl-6,7-dihydro-3H,5H-pyrrolo[2,3-f]benzimidazol-6-on
7,7-Dimethyl-2-pentafluorethyl-6,7-dihydro-3H,5H-pyrrolo[2,3-f]benzimidazol-6-on
7,7-Dimethyl-2-heptafluorpropyl-6,7-dihydro-3H,5H-pyrrolo[2,3-f]benzimidazol-6-on
7,7-Dimethyl-2-ethylmercapto-6,7-dihydro-3H,5H-pyrrolo[2,3-f]benzimidazol-6-on
7,7-Dimethyl-2-propylmercapto-6,7-dihydro-3H,5H-pyrrolo[2,3-f]benzimidazol-6-on
7,7-Dimethyl-2-ethylcarbonylamino-6,7-dihydro-3H,5H-pyrrolo[2,3-f]benzimidazol-6-on
7,7-Dimethyl-2-propylcarbonylamino-6,7-dihydro-3H,5H-pyrrolo[2,3-f]benzimidazol-6-on
7,7-Dimethyl-2-(3-pyridyl-carbonylamino)-6,7-dihydro-3H,5H-pyrrolo[2,3-f]benzimidazol-6-on
7,7-Dimethyl-2-cyclopentenyl-6,7-dihydro-3H,5H-pyrrolo[2,3-f]benzimidazol-6-on
2,7,7-Trimethyl-6,7-dihydro-3H,5H-pyrrolo[2,3-f]benzimidazol-6-thion
2,7-Dimethyl-6,7-dihydro-3H,5H-pyrrolo[2,3-f]benzimidazol-6-thion
1-Ethyl-2-hydroxy-7,7-dimethyl-6,7-dihydro-5H-pyrrolo[2,3-f]benzimidazol-6-on
1-Propyl-2-hydroxy-7,7-dimethyl-6,7-dihydro-5H-pyrrolo[2,3-f]benzimidazol-6-on
1-Methyl-2-hydroxy-6,7-dihydro-5H-pyrrolo[2,3-f]benzimidazol-6-on

1-Methyl-2-hydroxy-7-ethoxycarbonyl-7-methyl-6,7-dihydro-5H-pyrrolo[2,3-f]benzimidazol-6-on

Beispiel 1

7,7-Dimethyl-6,7-dihydro-3H,5H-pyrrolo[2,3-f]benzimidazol-6-on

288 mg (1.51 mmol) 5,6-Diamino-3,3-dimethyl-indolin-2-on und 66 mg (1.43 mmol) Ameisensaeure wurden eine Stunde auf 100° C erhitzt. Nach Zugabe von weiteren 24 mg (0.52 mmol) Ameisensaeure wurden weitere 60 Minuten auf 100° C erhitzt. Danach wurde mit je 0.5 ml Ether viermal angerieben und abgesaugt.
Man erhielt 277 mg (91 %) helle Kristalle mit Schmp. 278-280° C.

Beispiel 2

7,7-Dimethyl-2-cyclohexyl-6,7-dihydro-3H,5H-pyrrolo[2,3-f]benzimidazol-6-on

Analog Beispiel 1 erhielt man aus 960 mg (5.0 mmol) 5,6-Diamino-3,3-dimethyl-indolin-2-on und 640 mg (5.0 mmol) Cyclohexancarbonsaeure nach 1.5 Stunden bei 240° C 1.37 g Rohprodukt. 300 mg davon reinigte man ueber eine Kieselgelsaeule (Dichlormethan/Methanol = 9:1, v/v). Nach Entfernung des Loesungsmittels im Vakuum erhielt man ein gruenliches Oel, das mit Ether kristallisierte. Man erhielt 192 mg (53 %) der Titelverbindung als hellbraune Kristalle mit Schmp. 315-318° C.

Beispiel 3

2'-Propyl-spiro[cyclopentan-1,7'-6',7'-dihydro-3'H,5'H-pyrrolo[2',3'-f]benzimidazol]-6'-on

Analog Beispiel 1 erhitzte man eine Loesung von 6.0 g (27.6 mmol) 5',6'-Diamino-spiro[cyclopentan-1,3'-2',3'-dihydroindol]-2'-on, 5.4 ml (33.0 mmol) Buttersaeureanhydrid und 60 ml konzentrierte Salzsaeure in 250 ml Methanol 15 Stunden unter Rueckfluß. Die Loesung wurde im Vakuum zur Trockene eingeengt und der Rueckstand ueber eine Kieselgelsaeule (Laenge 70 cm, Durchmesser 6 cm, Laufmittel: Dichlormethan: methanolische Ammoniakloesung = 9:1) getrennt. Die entsprechenden Fraktionen wurden im Vakuum zur Trockene eingeengt und aus Methanol unter Zusatz von Aktivkohle umkristallisiert. Man erhielt 3.0 g (41 %) der Titelverbindung mit dem Schmelzpunkt 332-335° C.

Beispiel 4

7,7-Dimethyl-2-(2-propyl)-6,7-dihydro-3H,5H-pyrrolo[2,3-f]benzimidazol-6-on

a) 1.65 g (7.5 mmol) 6-Amino-5-nitro-3,3-dimethyl-indolin-2-on und 2.42 g Isobuttersaeureanhydrid in 15 ml Pyridin wurden eine Stunde auf 50° C gehalten. Nach einer Stunde wurden 1.2 g Isobuttersaeureanhydrid zugegeben und eine Stunde auf 50° C gehalten. Das Loesungsmittel wurde im Hochvakuum entfernt und der Rueckstand in 30 ml Dichlormethan geloest. Man gab 30 ml Eiswasser zu und neutralisierte mit $NaHCO_3$ (pH = 7-8). Man extrahierte viermal mit Dichlormethan, trocknete die vereinigten Dichlormethanphasen und behandelte mit Aktivkohle. Nach Filtration und Entfernung des Loesungsmittels wurde der Rueckstand mit Ether angerieben. Man erhielt 1.1 g (50 %) 6-Isobuttersaeureamido-5-nitro-3,3-dimethyl-indolin-2-on mit Schmp. 138-140° C.

b) Der obige Rueckstand wurde in 200 ml Methanol mit 400 mg 10 % Palladium auf Aktivkohle eine Stunde lang hydriert. Nach Filtration und Entfernung des Loesungsmittels im Vakuum erhielt man 0.8 g 5-Amino-6-isobuttersaeureamido-3,3-dimethyl-indolin-2-on als amorphe Substanz, die ohne weitere Reinigung eingesetzt wurde.

c) Der Rueckstand von b) wurde in 8 ml Isobuttersaeure suspendiert und eine Stunde bei 100° C geruehrt. Man dampfte im Hochvakuum ein, versetzte den Rueckstand mit 50 ml Eiswasser und brachte mit waessriger Ammoniakloesung auf pH = 8. Man extrahierte mit Essigester, trocknete und behandelte mit Aktivkohle. Man engte auf etwa 10 ml ein und saugte nach 30 min die ausgefallenen Kristalle ab. Man erhielt 0.37 g (40 %) der Titelverbindung mit dem Schmp. 282-284° C.

Beispiel 5

2-(1-Propyl)-7,7-dimethyl-6,7-dihydro-3H,5H-pyrrolo[2,3-f]benzimidazol-6-on

Nach dem Verfahren von Beispiel 5 erhielt man aus 0.7 g (2.7 mmol) 5-Amino-6-n-propylcarbonylamino-3,3,-dimethyl-indolin-2-on und 7 ml Buttersaeure nach einer Stunde bei 100°C 0.4 g (61 %) der Titelverbindung mit dem Schmelzpunkt 284-285°C.

Beispiel 6

7,7-Dimethyl-2-trifluormethyl-6,7-dihydro-3H,5H-pyrrolo[2,3-f]benzimidazol-6-on

Analog Beispiel 5 wurden 4.50 g (0.014 mol) 6-Trifluoracetamido-5-nitro-3,3-dimethyl-indolin-2-on in 500 ml Ethanol in Gegenwart von 0.5 g 10 % Palladium auf Aktivkohle bei Normaldruck und Raumtemperatur hydriert. Nach Filtration wurde das Filtrat im Vakuum zur Trockene eingeengt. Der Rueckstand wurde in Dichlormethan/Essigester geloest und mit Aktivkohle behandelt. Nach Filtration wurde im Vakuum zur Trockene eingeengt und der Rueckstand aus Essigester kristallisiert. Man erhielt 3.8 g (95 %) der Titelverbindung als Monohydrat mit dem Schmp. 175-177°C.

Beispiel 7

2,7,7-Trimethyl-6,7-dihydro-3H,5H-pyrrolo[2,3-f]benzimidazol-6-on

Analog dem Beispiel 5 suspendierte man 5.00 g (0.019 mol) 6-Acetamido-5-nitro-3,3-dimethyl-indolin-2-on in 900 ml Eisessig und hydrierte in Gegenwart von 0.5 g Platindioxid bei Normaldruck und Raumtemperatur. Nachdem 1.4 Liter Wasserstoff aufgenommen waren wurde filtriert und das Filtrat im Vakuum zur Trockene eingeengt. Der Rueckstand wurde in Wasser ausgenommen und mit 2 N Ammoniakloesung neutralisiert. Der ausgefallene Niederschlag wurde abgesaugt und aus Essigester umkristallisiert. Man erhielt 2.75 g (67 %) der Titelverbindung mit dem Schmelzpunkt >300°C.

Beispiel 8

2-Propyl-7-ethoxycarbonyl-7-methyl-6,7-dihydro-3H,5H-pyrrolo[2,3-f]benzimidazol-6-on

Analog dem Beispiel 5 suspendierte man 1.40 g (4.0 mmol) 6-Propylcarbonylamino-5-nitro-3-ethoxycarbonyl-3-methyl-indolin-2-on in 140 ml Ethanol und hydrierte in Gegenwart von 0.1 g 10 % Palladium auf Kohle bei Normaldruck und Raumtemperatur. Nachdem 270 ml Wasserstoff aufgenommen waren wurde der Katalysator abgesaugt und das Filtrat zur Trockene eingeengt. Den Rueckstand ruehrte man eine Stunde bei 60°C in 10 ml Eisessig, man destillierte den Eisessig ab, nahm in Wasser auf und stellte mit 2 N Natronlauge neutral. Man schuettelte dreimal mit Dichlormethan aus, trocknete die vereinigten Dichlormethanphasen ueber Natriumsulfat, filtrierte und engte das Filtrat zur Trockene ein. Nach Kristallisation des Rueckstandes aus Isopropanol erhielt man 0.4 g (33 %) der Titelverbindung mit dem Schmelzpunkt 258-261°C.

Beispiel 9

7,7-Dimethyl-2-(iso-propenyl)-6,7-dihydro-3H,5H-pyrrolo[2,3-f]benzimidazol-6-on

3.0 g (15.7 mmol) 5,6-Diamino-3,3-dimethyl-indolin-2-on und 1.37 g (15.9 mmol) Methacrylsaeure wurden in 28 g Polyphosphorsaeure 30 min auf 200°C erhitzt. Danach versetzte man mit 40 ml Eiswasser. Die erhaltene braune Suspension wurde unter Eiskuehlung mit 45 ml konz. Ammoniakloesung auf pH = 8 gestellt. Die Substanz wurde abgesaugt und mit wenig Wasser gewaschen. Man erhielt 3.74 g Rohprodukt, das man in 5 ml DMSO loeste, mit 15 ml Methanol verduennte und ueber eine Kieselgelsaeule (750 g Gel) reinigte. (Dichlormethan/Methanol = 10:1, v/v). Man erhielt 0.6 g (14 %) der Titelverbindung mit dem Schmp. 270-273°C.

Beispiel 10

2-(1-Propenyl)-7,7-dimethyl-6,7-dihydro-3H,5H-pyrrolo[2,3-f]benzimidazol-6-on

Analog dem Beispiel 11 erhitzte man 0.95 g 5,6-Diamino-3,3-dimethyl-indolin-2-on und 0.43 g Crotonsaeure mit 10 ml Polyphosphorsaeure 30 Minuten auf 170° C (Badtemperatur). Man loeste das abgekuehlte Reaktionsgut in 40 ml Wasser, neutralisierte mit konzentrierter waessriger Ammoniakloesung, saugte das Ungeloeste ab, wusch mit Wasser und loeste es in 100 ml heißem Ethanol. Nach Behandeln mit Aktivkohle dampfte man das heiße Filtrat im Vakuum ein, rieb den Eindampfrueckstand mit Ether an und erhielt so 0.7 g der Titelverbindung mit dem Schmelzpunkt 252-254° C, der 0.5 mol Wasser pro mol Substanz anhaftete.

Beispiel 11

2-n-Hexyl-7,7-dimethyl-6,7-dihydro-3H,5H-pyrrolo[2,3-f]benzimidazol-6-on

Analog dem Beispiel 11 erhielt man aus 0.48 g 5,6-Diamino-3,3-dimethyl-indolin-2-on und 0.36 g Oenanthsaeure nach dem Erhitzen mit 4.5 g Polyphosphorsaeure (30 min, 200° C Badtemperatur) 0.7 g Rohprodukt, das man in 35 ml Ethanol bei 50° C loeste, mit Aktivkohle behandelte und das klare Filtrat im Vakuum eindampfte. Der Eindampfrueckstand ergab nach Anreiben mit Ether 0.37 g der Titelverbindung als Monohydrat mit dem Schmelzpunkt 233-234° C.

Beispiel 12

7,7-Dimethyl-2-(1-cyclopenten-1-yl)-6,7-dihydro-3H,5H-pyrrolo[2,3-f]benzimidazol-6-on

Analog dem Beispiel 11 wurde eine Mischung von 0.48 g 5,6-Diamino-3,3-dimethyl-indolin-2-on und 0.31 g (1-Cyclopenten-1-yl)carbonsaeure mit warmer Polyphosphorsaeure (4.5 g) gut durchgeruehrt und dann 30 Minuten in einem Bad von 200° C unter Ruehren gehalten. Nach dem Erkalten wurde der Ansatz mit Eiswasser durchgerieben, mit konz. waess. Ammoniak auf ca. pH 7-8 gestellt, das Ungeloeste abgesaugt, mit Wasser gewaschen und getrocknet. So fielen 0.51 g Rohprodukt an. Dieseswurde wiederholt mit jeweils 6 ml Methanol angerieben, wobei 0.25 g Substanz ungeloest verblieben. Man rieb dieses Produkt 3 mal mit je 6 ml eines Gemisches von Methylenchlorid/$CH_3OH$ 1:1 durch und erhielt so 0.14 g der gewuenschten Titelverbindung, welcher pro mol noch 2/3 mol Methanol und 1 mol $H_2O$ anhafteten. Fp. 233/235° C.

Aus den eingedampften Methanolfiltraten erhielt man nach analoger Reinigung noch weitere 0.1 g der gewuenschten Substanz.

Beispiel 13

2-Cyclopropyl-7,7-dimethyl-6,7-dihydro-3H,5H-pyrrolo[2,3-f]benzimidazol-6-on

Analog dem Beispiel 11 erhielt man aus 0.95 g 5,6-Diamino-3,3-dimethyl-indolin-2-on und 0.45 g Cyclopropancarbonsaeure nach dem Erhitzen mit 10 g Polyphosphorsaeure (1 h, 150° C Badtemperatur) 1.18 g Rohprodukt, welches man wiederholt mit Ethanol auskochte. Aus den vereinigten Ethanolextrakten erhielt man nach Behandeln mit Aktivkohle, Eindampfen des heißen, klaren Filtrats im Vakuum und Anreiben des Eindampfrueckstandes mit Ether 0.52 g der Titelverbindung mit dem Schmelzpunkt 266-268° C, welcher 0.75 mol Wasser pro mol Substanz anhaftete.

Beispiel 14

2-(1,1-Dimethyl-ethyl)-7,7-dimethyl-6,7-dihydro-3H,5H-pyrrolo[2,3-f]benzimidazol-6-on

Analog dem Beispiel 11 erhitzte man 0.286 g 5,6-Diamino-3,3-dimethyl-indolin-2-on und 0.18 g Pivalinsaeure mit 3 g Polyphosphorsaeure in einem vorgeheitzten Bad 30 min auf 200° C, gab dann nochmals 0.18 g Pivalinsaeure zu und erhitzte weitere 30 min auf 200° C und wiederholte diese Prozedur noch dreimal, so daß insgesamt 0.9 g Pivalinsaeure und 2.5 Stunden Reaktionszeit in Anwendung kamen. Nach dem Abkuehlen loeste man in ca. 9 ml Wasser, stellte mit konz. waessrigem Ammoniak auf ca. pH 7 bis 8, saugte die erhaltene Faellung ab, wusch mit Wasser und erhielt so 0.28 g der Titelverbindung mit dem Schmelzpunkt >300° C. Zur weiteren Reinigung wurde in wenig Ethanol heiß geloest, die truebe Loesung mit Kohle behandelt, das heiße, klare Filtrat im Vakuum eingedampft und der Eindampfrueckstand mit Ether angerieben. Ein mol der Titelverbindung enthielt 1 mol Wasser adiert. Schmelzpunkt >300° C.

Beispiel 15

7,7-Dimethyl-2-hydroxy-6,7-dihydro-3H,5H-pyrrolo[2,3-f]benzimidazol-6-on

In eine Loesung von 4.00 g (0.021 mol) 5,6-Diamino-3,3,-dimethylindolin-2-on in 60 ml 2 N HCl leitete man bei Raumtemperatur eine Stunde lang Phosgen ein. Danach leitete man zehn Minuten lang Stickstoff durch die Loesung und ließ dann 15 Stunden stehen. Der Niederschlag wurde filtriert und aus Methanol kristallisiert. Man erhielt 2.80 g (62 %) der Titelverbindung mit Schmp. >300 ° C.

Beispiel 16

Analog dem Beispiel 17 erhielt man aus 4.80 g (0.019 mol) 5,6-Diamino-3-methyl-indolin-2-on 3.00 g (77 %) 7-Methyl-2-hydroxy-6,7-dihydro-3H,5H-pyrrolo[2,3-f]benzimidazol-6-on mit dem Schmelzpunkt >300 ° C nach Kristallisation aus Methanol.

Beispiel 17

Analog dem Beispiel 17 erhielt man aus 2.50 g (0.013 mol) 5,6-Diamino-3-ethyl-indolin-2-on 1.80 g (64 %) 7-Ethyl-2-hydroxy-6,7-dihydro-3H,5H-pyrrolo[2,3-f]benzimidazol-6-on mit dem Schmelzpunkt >300 ° C nach Kristallisation aus Pyridin.

Beispiel 18

Analog dem Beispiel 17 erhielt man aus 12.0 g (0.063 mol) 5,6-Diamino-3,3-dimethylindolin-2-on und 25 ml Thiophosgen 2.75 g (19 %) 7,7-Dimethyl-2-mercapto-6,7-dihydro-3H,5H-pyrrolo[2,3-f]benzimidazol-2-on mit Schmp. >300 ° C (nach Reinigung ueber eine Kieselgelsaeule mit Dichlormethan/Methanol = 7:3 als Laufmittel und anschließender Kristallisation aus Methanol)

Beispiel 19

7,7-Dimethyl-2-amino-6,7-dihydro-3H,5H-pyrrolo[2,3-f]benzimidazol-6-on

3.00 g (0.016 mol) 5,6-Diamino-3,3-dimethylindolin-2-on wurde in 100 ml Ethanol suspendiert und bei Raumtemperatur mit 1.82 g (0.017 mol) Bromcyan versetzt und 2 Stunden geruehrt. Man entfernte das Loesungsmittel im Vakuum. Den Rueckstand loeste man in Ethanol, behandelte mit Aktivkohle und faellt durch Zugabe von Aceton. Man kristallisierte aus Aceton und wenig Ethanol und erhielt 1.05 g (22 %) der Titelverbindung als Hydrobromid mit dem Schmp.: 300-305 ° C.

Beispiel 20

2-(4-Pyridylcarbonylamino)-7,7-dimethyl-6,7-dihydro-3H,5H-pyrrolo[2,3-f]benzimidazol-6-on

2.7 g (9.1 mmol) der in Beispiel 23 dargestellten Verbindung und 3.8 ml Triethylamin in 50 ml Pyridin versetzt man portionsweise bei 0 ° C mit 1.62 g (9.1 mmol) Isonicotinsaeurechlorid-Hydrochlorid. Man erhitzte fuenf Stunden auf 50 ° C, destillierte das Pyridin ab und digerierte den Rueckstand mit Wasser. Nach Kristallisation aus Ethanol erhielt man 1.5 g der Titelverbindung mit dem Schmelzpunkt >300 ° C.

Beispiel 21

7,7-Dimethyl-2-methylmercapto-6,7-dihydro-3H,5H-pyrrolo [2,3-f]benzimidazol-6-on

1.35 g (5.79 mmol) der in Beispiel 20 dargestellten Verbindung und 0.8 g Kaliumcarbonat suspendierte man in 120 ml Aceton. Nach 20 min gab man unter Eiskuehlung tropfenweise 0.4 ml Jodmethan zu. Nach 40 h bei Raumtemperatur entfernte man das Loesungsmittel im Vakuum und digerierte den Rueckstand mit Wasser. Nach Reinigung ueber eine Kieselgelsaeule (Dichlormethan/Methanol 7:3) und Kristallisation aus Methanol erhielt man 0.7 g (49 %) der Titelverbindung mit dem Schmelzpunkt 248-250 ° C.

Beispiel 22

2-Propyl-7-methyl-6,7-dihydro-3H,5H-pyrrolo[2,3-f]benzimidazol-6-on

1.50 g (4.9 mmol) der in Beispiel 10 dargestellten Verbindung ruehrte man in 30 ml 60 % Schwefelsaeure eine Stunde bei 60° C Badtemperatur, goß auf Eis, neutralisierte mit 2 N waessriger Ammoniakloesung, saugte die ausgefallene Substanz ab und kristallisierte den Rueckstand aus Essigester/Methanol. Man erhielt 0.70 g (62 %) der Titelverbindung mit dem Schmelzpunkt 235-238° C.

**Patentansprüche**

1. Verbindungen der allgemeinen Formel I

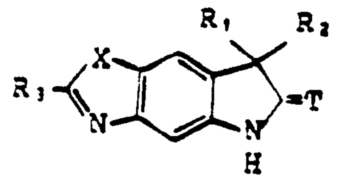

(I)

in welcher
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, ein $C_1$-$C_6$ Alkyl-, $C_2$-$C_6$-Alkenyl- oder Cyangruppe, eine durch eine Hydroxy-, $C_1$-$C_6$-Alkyl-, $C_1$-$C_6$- Alkoxy-, Amino-, $C_1$-$C_6$-Alkylamino-, Di-$C_1$-$C_6$-alkylamino- oder Hydrazinogruppe substituierte Carbonylgruppe bedeutet oder mit $R_1$ zusammen eine $c_3$-$C_7$-Cycloalkylengruppe darstellt oder $R_1$ und $R_2$ zusammen eine $C_3$-$C_7$-Alkyliden- oder $C_3$-$C_7$-Cycloalkylidengruppe bilden,
T Sauerstoff oder Schwefel bedeutet,
$R_3$ ein Wasserstoffatom, die Hydroxygruppe, eine Mercapto-, $C_1$-$C_6$-Alkylmercapto-, eine Amino-, Pyridylcarbonylamino-, $C_1$-$C_6$-Alkylcarbonylaminogruppe, eine $C_1$-$C_6$-Alkyl-, $C_3$-$C_7$-Cycloalkyl-, $C_2$-$C_6$-Alkenyl-, $C_3$-$C_7$-Cycloalkenyl-, $C_2$-$c_6$-Alkinyl- oder Halogen-$C_1$-$C_6$-alkylgruppe bedeutet,
X die Gruppe -NH- darstellt,
deren Tautomere und deren physiologisch verträgliche Salze anorganischer und organischer Säuren.

2. Verbindungen der allgemeinen Formel I gemäß Anspruch 1, in der
$R_1$ und $R_2$ gleich sind und die Methyl- oder Ethylgruppe darstellen,
$R_1$ und $R_2$ verschieden sind und Wasserstoff, die Methyl-, Ethyl-, Isopropyl-, Cyclopentyl-, Cyan-, Acetyl-, Methoxycarbonyl-, Ethoxycarbonyl-, Aminocarbonyl- und Hydrazinocarbonylgruppe bedeuten,
$R_1$ und $R_2$ einen Spirocyclopentylring darstellen, wenn $R_1$ und $R_2$ mit dem C-Atom, an das sie gebunden sind, einen $C_3$-$C_7$-Cycloalkylring bilden,
$R_3$ ein Wasserstoffatom, die Methyl-, Ethyl-, Propyl-, Isopropyl-, Butyl-, Tert.-butyl-, Pentyl-, Hexyl-, Propenyl-Isopropenyl-, Cyclopropyl-, Cyclopentyl-, Cyclohexyl-, Trifuormethyl-, Hydroxy-, Mercapto-, Methylmercapto-, Amino-Acetylamino- oder Pyridylcabonylaminogruppe darstellt,
T ein Sauerstoffatom darstellt,
deren Tautomere und deren physiologisch verträglichen Salze anorganischer und organischer Säuren.

3. Verbindungen der allgemeinen Formel I gemäß Anspruch 1 oder 2,
in der

| | |
|---|---|
| $R_1$ | die Methyl- oder Ethylgruppe darstellt, |
| $R_2$ | Wasserstoff oder die Methyl-, Ethyl-, Methoxycarbonyl- oder Ethoxycarbonylgruppe bedeutet oder |
| $R_1$ und $R_2$ | zusammen mit dem C-Atom, an das sie gebunden sind, eine Spirocyclopentyl- oder Spirocyclohexylgruppe darstellen, |
| $R_3$ | ein Wasserstoffatom, die Methyl-, Ethyl-, Propyl-, Hexyl-, Isopropyl-, Propenyl-, Isopropenyl-, Tert.-butyl-, Cyclopropyl-, Cyclohexyl-, Cyclopentenyl-, Trifluormethyl-, Hydroxy-, Mercapto-, Methylmercapto-, Amino- oder Pyridylcarbonylaminogruppe bedeutet, |
| T | ein Sauerstoffatom darstellt, |

deren Tautomere und deren physiologisch verträglichen Salze anorganische und organische Säuren.

4. Verfahren zur Herstellung von Verbindungen der allgemeinen Formel I gemäß Anspruch 1, 2 oder 3, dadurch gekennzeichnet, daß man

A) Verbindungen der Formel IX

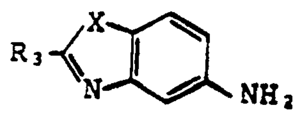

(IX)

in der $R_3$ die angegebene Bedeutung hat,
mit Bisulfit-Additions-Verbindungen von entsprechenden Ketonen (Hinsberg-Synthese) oder über ein entsprechendes Hydrazid (Brunner-Synthese) oder über ein entsprechendes Amid (Stolle- Synthese) cyclisiert
oder

B) Verbindungen der Formel VII

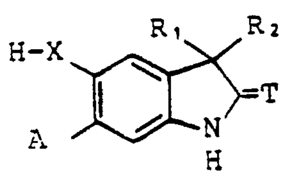

(VII')

in der $R_1$, $R_2$, X und T die angegebenen Bedeutungen haben und A eine $NH_2$ oder eine $NO_2$-Gruppe darstellt,
mit einer Verbindung der Formel VIII

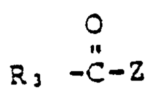

(VIII)

in der Z Wasserstoff, eine Hydroxygruppe oder einen leicht abspaltbaren Rest bedeutet,
umsetzt und cyclisiert,
um im Fall, daß A eine $NO_2$-Gruppe darstellt,
reduziert und anschließend cyclisiert
oder

C) für den Fall, daß $R_3$ eine Hydroxy, Mercapto oder Aminogruppe bedeutet,
Verbindungen der Formel VII

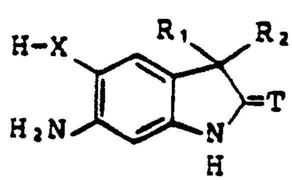

(VII)

mit einer eine Carbonyl-, Thiocarbonyl- oder Iminogruppe enthaltenen Verbindung cyclisiert
oder
D) eine Verbindung der Formel II

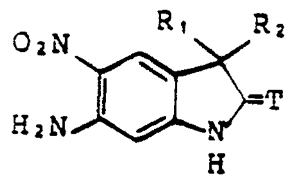

(II)

mit einer Verbindung der Formel III

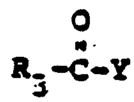

(III)

in der $R_3$ die angegebene Bedeutung hat und Y eine Hydroxy oder eine leicht abspaltbare Gruppe darstellt,
acyliert und cyclisiert
und anschließend gewünschtenfalls erhaltene Verbindungen der Formel I in andere Verbindungen der Formel I umwandelt sowie gegebenenfalls die Verbindungen in pharmakologisch verträgliche Salze überführt.

5. Verwendung von Verbindungen der Formel I gemäß Anspruch 1, 2 oder 3 zur Herstellung von Arzneimitteln zur Behandlung oder Prophylaxe von Herz- und Kreislauferkrankungen.

6. Arzneimittel, enthaltend eine oder mehrere Verbindungen der Formel I gemäß Anspruch 1, 2 oder 3 neben üblichen pharmakologischen Träger- und/oder Hilfsstoffen.

7. Verbindungen der Formel I gemäß Anspruch 1, 2 oder 3 zur Behandlung und/oder Prophylaxe von Herz- und Kreislauferkrankungen.

8. Verfahren zur Herstellung von Arzneimitteln gemäß Anspruch 6, dadurch gekennzeichnet, daß man Verbindungen der Formel I mit üblichen pharmakologischen Träger- und/oder Hilfsstoffen vermischt und zu Arzneimitteln verarbeitet.

## Claims

1. Compounds of the general formula I

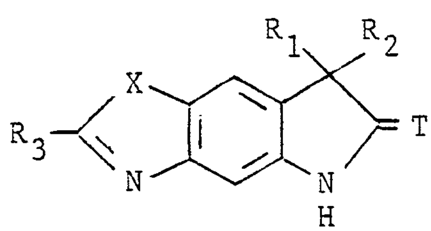

(I)

in which $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino group or, together with $R_1$, represents a $C_3$-$C_7$-cycloalkylene group or $R_1$ and $R_2$ together form a $C_3$-$C_7$-alkylidene or $C_3$-$C_7$-cycloalkylidene group, T signifies oxygen or sulphur, $R_3$ signifies a hydrogen atom, the hydroxyl group, a mercapto, $C_1$-$C_6$-alkylmercapto, an amino, pyridylcarbonylamino, $C_1$-$C_6$-alkylcarbonylamino, a $C_1$-$C_6$-alkyl, $C_3$-$C_7$-cycloalkyl, $C_2$-$C_6$-alkenyl, $C_3$-$C_7$-cycloalkenyl, $C_2$-$C_6$-alkynyl or halogeno-$C_1$-$C_6$-alkyl group, X represents the group -NH-, their tautomers and their physiologically acceptable salts of inorganic and organic acids.

2. Compounds of the general formula I according to claim 1, in which $R_1$ and $R_2$ are the same and represent the methyl or ethyl group, $R_1$ and $R_2$ are different and signify hydrogen, the methyl, ethyl, isopropyl, cyclopentyl, cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$ form a $C_3$-$C_7$-cycloalkyl ring with the C-atom to which they are attached, $R_3$ represents a hydrogen atom, the methyl, ethyl, propyl, isopropyl, butyl, tert.-butyl, pentyl, hexyl, propenyl, isopropenyl, cyclopropyl, cyclopentyl, cyclohexyl, trifluoromethyl, hydroxyl, mercapto, methylmercapto, amino, acetylamino or pyridylcarbonylamino group, T represents an oxygen atom, their tautomers and their physiologically acceptable salts of inorganic and organic acids.

3. Compounds of general formula I according to claim 1 or 2, in which $R_1$ represents the methyl or ethyl group, $R_2$ signifies hydrogen or the methyl, ethyl, methoxycarbonyl or ethoxycarbonyl group or $R_1$ and $R_2$, together with the C-atom to which they are attached, represent a spirocyclopentyl or spirocyclohexyl group, $R_3$ signifies a hydrogen atom, the methyl, ethyl, propyl, hexyl, isopropyl, propenyl, isopropenyl, tert.-butyl, cyclopropyl, cyclohexyl, cyclopentenyl, trifluoromethyl, hydroxyl, mercapto, methylmercapto, amino or pyridylcarbonylamino group, T represents an oxygen atom, their tautomers and their physiologically acceptable salts of inorganic and organic acids.

4. Process for the preparation of compounds of the general formula I according to claim 1, 2 or 3, characterised in that one

A) cyclises compounds of the formula IX

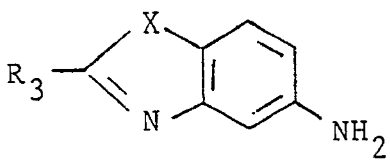

(IX)

in which $R_3$ has the given meaning, with bisulphite addition compounds of corresponding ketones (Hinsberg synthesis) or via an appropriate hydrazide (Brunner synthesis) or via a corresponding amide (Stolle synthesis) or

B) reacts compounds of the formula VII

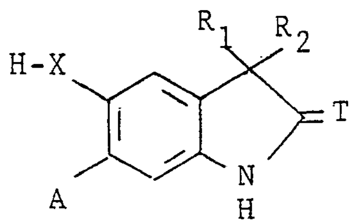

(VII)

in which $R_1$, $R_2$, X and T have the given meanings and A represents an $NH_2$ or an $NO_2$ group, with a compound of the formula VIII

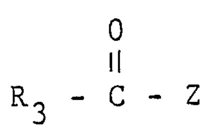

(VIII)

in which Z signifies hydrogen, a hydroxyl group or a residue which can easily be split off, and cyclises or, in the case that A represents an $NO_2$ group, reduces and subsequently cyclises, or

C) for the case that $R_3$ signifies a hydroxyl, mercapto or amino group, cyclises compounds of the formula VII

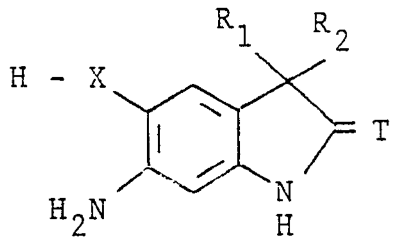

(VII)

with a carbonyl, thiocarbonyl or imino group-containing compound or
D) acylates a compound of the formula II

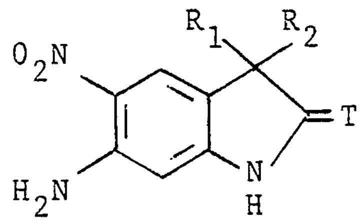

with a compound of the formula III

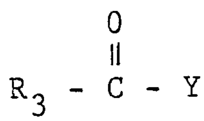

(III)

in which $R_3$ has the given meaning and Y represents a hydroxyl group or a group which is easily split off, and cyclises
and subsequently, if desired, converts compounds obtained of the formula I into other compounds of the formula I, as well as possibly converts the compounds into pharmacologically acceptable salts.

5. Use of compounds of the formula I according to claim 1, 2 or 3 for the preparation of medicaments for the treatment or prophylaxis of heart and circulatory diseases.

6. Medicaments containing one or more compounds of the formula I according to claim 1, 2 or 3, besides usual pharmacological carrier and/or adjuvant materials.

7. Compounds of the formula I according to claim 1, 2 or 3 for the treatment and/or prophylaxis of heart and circulatory diseases.

8. Process for the preparation of medicaments according to claim 6, characterised in that one mixes compounds of the formula I with usual pharmacological carrier and/or adjuvant materials and works up to medicaments.

## Revendications

1. Composés de formule générale I

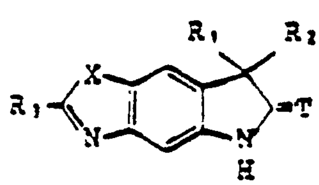

(I)

dans laquelle

$R_1$ représente un atome d'hydrogène, un groupe alkyle en $C_1$-$C_6$, alcényle en $C_2$-$C_6$ ou cycloalkyle en $C_3$-$C_7$,

$R_2$ représente un atome d'hydrogène, un groupe alkyle en $C_1$ -$C_6$, alcényle en $C_2$-$C_6$ ou cyano, ou un groupe carbonyle substitué par un groupe hydroxy, alkyle en $C_1$-$C_6$, alcoxy en $C_1$-$C_6$, amino, alkylamino en $C_1$-$C_6$, dialkylamino en $C_1$-$C_6$ ou hydrazino, ou bien, conjointement avec $R_1$, représente un groupe cycloalkylène en $C_3$-$C_7$ ou bien $R_1$ et $R_2$ forment ensemble un groupe alkylidène en $C_3$-$C_7$ ou cycloalkylidène en $C_3$-$C_7$,

T représente l'oxygène ou le soufre,

$R_3$ représente un atome d'hydrogène, le groupe hydroxy, un groupe mercapto, alkylmercapto en $C_1$-$C_6$, amino, pyridylcarbonylamino, alkylcarbonylamino en $C_1$-$C_6$, alkyle en $C_1$-$C_6$, cycloalkyle en $C_3$-$C_7$, alcényle en $C_2$-$C_6$, cycloalcényle en $C_3$-$C_7$, alcinyle en $C_2$-$C_6$ ou halogénoalkyle en $C_1$-$C_6$,

X représente le groupe -NH-,

leurs tautomères, et leurs sels physiologiquement acceptables formés avec des acides minéraux et organiques.

2. Composés de formule générale I selon la revendication 1, dans lesquels

$R_1$ et $R_2$ sont identiques et représentent le groupe méthyle ou éthyle,

$R_1$ et $R_2$ sont différents et représentent l'hydrogène, les groupes méthyle, éthyle, isopropyle, cyclopentyle, cyano, acétyle, méthoxy-carbonyle, éthoxycarbonyle, aminocarbonyle et hydrazinocarbonyle,

$R_1$ et $R_2$ représentent un cyle spirocyclopentyle lorsque $R_1$ et $R_2$ forment, avec l'atome de C auquel ils sont liés, un cycle cycloalkyle en $C_3$-$C_7$,

$R_3$ représente un atome d'hydrogène, le groupe méthyle, éthyle, propyle, isopropyle, butyle, tert.-butyle, pentyle, hexyle, propényle, isopropényle, cyclopropyle, cyclopentyle, cyclohexyle, trifluorométhyle, hydroxy-, mercapto-, méthylmercapto-, amino-, acétylamino- ou pyridiylcarbonylamino,

T représente une atome d'oxygène,

leurs tautomères, et leurs sels physiologiquement acceptables formés avec des acides minéraux et organiques.

3. Composés de formule générale I selon les revendications 1 ou 2,
dans lesquels

$R_1$ représente le groupe méthyle ou éthyle,

$R_2$ représente l'hydrogène ou le groupe méthyle, éthyle, méthoxycarbonyle ou éthoxycarbonyle ou

$R_1$ et $R_2$ forment ensemble avec l'atome de C auquel ils sont liés un groupe spirocyclopentyle ou spirocyclohexyle,

$R_3$ représente un atome d'hydrogène, le groupe méthyle, éthyle, propyle, hexyle, isopropyle, propényle, isopropényle, tert.-butyle, cyclopropyle, cyclohexyle, cyclopentényle, trifluorométhyle, hydroxy-, mercapto-, méthylmercapto-, amino- ou pyridylcarbonylamino,

T représente un atome d'oxygène,

leurs tautomères, et leurs sels physiologiquement acceptables formés avec des acides minéraux et organiques.

4. Procédé de préparation des composés de formule générale I selon la revendication 1, 2 ou 3, caractérisé en ce que

A) on cyclise des composés de formule IX

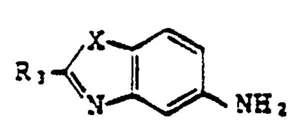

(IX)

dans laquelle $R_3$ a la signification mentionnée,

avec des composés d'addition bisulfitiques de cétones appropriées (synthèse de Hinsberg)
ou bien par l'intermédiaire d'un hydrazide approprié (synthèse de Brunner)
ou par l'intermédiaire d'un amide approprié (synthèse de Stolle)

ou

B) on fait réagir et on cyclise des composés de formule VII

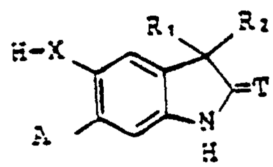

(VII')

dans laquelle $R_1$, $R_2$, X et T ont les significations mentionnées et A représente un groupe $NH_2$ ou $NO_2$,
avec un composé de formule VIII

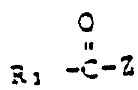

(VIII)

dans laquelle Z représente un atome d'hydrogène, un groupe hydroxy ou un reste facilement séparable,
dans le cas où A représente un groupe $NO_2$, on réduit et ensuite on cyclise,
ou
C) dans le cas où $R_3$ représente un groupe hydroxy, mercapto ou amino,
on cyclise des composés de formule VII

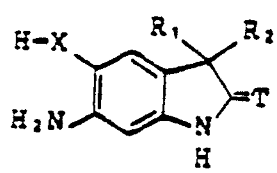

(VII)

avec un composé contenant un groupe carbonyle, thiocarbonyle ou imino
ou

D) on acyle et cyclise un composé de formule II

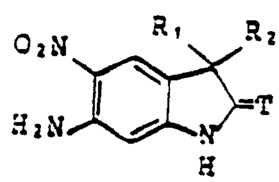

(II)

avec un composé de formule III

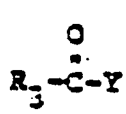

(III)

dans laquelle $R_3$ a la signification mentionnée et Y représente un groupe hydroxy ou un groupe

facilement séparable, et ensuite on transforme éventuellement les composés de formule I obtenus en d'autres composés de formule I et éventuellement on transforme ces composés en leurs sels pharmacologiquement acceptables.

**5.** Utilisation des composés de formule I selon la revendication 1, 2 ou 3 pour la préparation de médicaments destinés au traitement ou à la prophylaxie de maladies cardiovasculaires.

**6.** Médicaments contenant un ou plusieurs composés de formule I selon la revendication 1, 2 ou 3, en plus de supports et/ou d'adjuvants pharmaceutiques usuels.

**7.** Composé de formule I selon la revendication 1, 2 ou 3, destiné au traitement et/ou à la prophylaxie de maladies cardiovasculaires.

**8.** Procédé de préparation des médicaments selon la revendication 6, caractérisé en ce que l'on mélange les composés de formule I avec des supports et/ou adjuvants pharmacologiques usuels et on les transforme en médicaments.